# EUROPÄISCHE PATENTSCHRIFT

(11) **EP 2 814 430 B1**
(45) Veröffentlichungstag und Bekanntmachung des Hinweises auf die Patenterteilung: **13.12.2017**
(21) Anmeldenummer: 13705961.4
(22) Anmeldetag: 13.02.2013
(51) Int. Cl.: A61F 2/46, A61F 2/36

(54) **VERFAHREN ZUM PRÜFEN VON KERAMISCHEN KUGELKÖPFEN FÜR HÜFTGELENKSPROTHESEN**
METHOD FOR CHECKING CERAMIC BALL HEADS FOR HIP-JOINT PROSTHESES
PROCÉDÉ DE CONTRÔLE DE TÊTES SPHÉRIQUES EN CÉRAMIQUE DESTINÉES À DES PROTHÈSES DE HANCHE

(30) Priorität: 16.02.2012 DE 102012202372
(43) Veröffentlichungstag der Anmeldung: 24.12.2014
(73) Patentinhaber: CeramTec GmbH, 73207 Plochingen (DE)
(72) Erfinder: FLOHR, Markus, 73733 Esslingen (DE); UPMANN, Carsten, 70376 Stuttgart (DE); BERTMARING, Hendrik, 73732 Esslingen (DE)
(74) Vertreter: Uppena, Franz
(86) Internationale Anmeldenummer: PCT/EP2013/052890
(87) Internationale Veröffentlichungsnummer: WO 2013/120906

(56) Entgegenhaltungen:
- EP-A1- 1 449 501
- WO-A1-2009/068234
- DE-C2- 4 411 508
- JP-A- 2007 307 011

## Beschreibung

Die Erfindung betrifft ein Verfahren zum Prüfen von keramischen Kugelköpfen für Hüftgelenksprothesen, die über einen Aufnahmeraum mit einer konischen Seitenfläche mit einem Klemmkonuswinkel γ und einem Konuseingang verfügen und zur Prüfung Bereiche des Aufnahmeraums mit einem Druck beaufschlagt werden. Die erforderliche Mindestfestigkeit von modularen, keramischen Kugelköpfen wird über einen sogenannten Prooftest (100%-Prüfung) sichergestellt. Dabei wird der konische Bereich des Kugelkopfes hydraulisch belastet. Beschrieben ist dies in DE 44 11 508 C2. WO2009/068234 zeigt eine Testvorrichtung für ein künstliche Hüftgelenkkugeln, ebenso JP2007307011 und EP1449501. Bei speziellen Geometrien modularer Kugelköpfe kann es unter in-vivo relevanten, schrägen Belastungssituationen zu hohen Spannungen im Bereich des Konuseingangs kommen.

Nach heutigem Kenntnisstand gibt es noch kein Verfahren (Prooftest), welches den Konuseingang modularer Kugelköpfe prüft und im Rahmen einer 100%-Prüfung für diesen Bereich die erforderliche Mindestfestigkeit sicherstellt. 100%-Prüfung bedeutet, dass Kugelköpfe mit Fehlern im Prooftest zerbrechen, so dass nur einwandfreie Kugelköpfe den Prooftest überstehen.

Der Erfindung liegt die Aufgabe zugrunde, ein Verfahren nach dem Oberbegriff des Anspruchs 1 so zu verbessern, dass alle geprüften Kugelköpfe auch in-vivo relevanten, schrägen Belastungssituationen ohne Schaden zu nehmen funktionsbereit bleiben.

Erfindungsgemäß wird diese Aufgabe durch die Merkmale des Anspruchs 1 gelöst.

Das erfindungsgemäße Verfahren zum Prüfen von keramischen Kugelköpfen für Hüftgelenksprothesen, die über einen Aufnahmeraum mit einer konischen Seitenfläche mit einem Klemmkonuswinkel γ und einem Konuseingang verfügen und zur Prüfung Bereiche des Aufnahmeraums mit einem Druck beaufschlagt werden, zeichnet sich dadurch aus, dass ausschließlich der Bereich des Konuseingangs mit einer zur Längsachse des Kugelkopfes senkrecht verlaufenden radialen Kraft beauftragt wird. Hierdurch bleiben alle geprüften Kugelköpfe, auch in-vivo relevanten schrägen Belastungssituationen, ohne Schaden zu nehmen funktionsbereit.

Bevorzugt wird zum Prüfen eine konische Hülse mit einem Konuswinkel α in den Aufnahmeraum gedrückt, wobei der Konuswinkel α größer als der Klemmkonuswinkel γ ist. Hierdurch wird nur der Konuseingang belastet.

In einer Ausführungsform wird der Konuswinkel α im Bereich von 7° bis 30°, bevorzugt 18° gewählt und ist der Unterschied zwischen Klemmkonuswinkel γ und Konuswinkel α zwischen minimal 2° und maximal 25° zu wählen.

Es hat sich herausgestellt, dass bevorzugt die Hülse auf ihrer Umfangsfläche geschlossen ist und bevorzugt aus einer Kupferlegierung, bevorzugt Messing besteht.

Bevorzugt wird ein konischer Druckstempel mit einem mit der Hülse gemeinsamen Konuswinkel α axial mit einer Kraft F in die Hülse gedrückt.

In einer Ausführungsform wird sichergestellt, dass die Reibung zwischen Hülse und Druckstempel niedriger ist als die Reibung zwischen Hülse und Kugelkopf.

Bevorzugt ist der Druckstempel aus Stahl gefertigt und ist bevorzugt oberflächengehärtet.

In einer Ausführungsform wird zum Prüfen die Innenoberfläche der Hülse mit einem hydraulischen Druck beaufschlagt.

Eine Vorrichtung zur Durchführung des erfindungsgemäßen Verfahrens zeichnet sich dadurch aus, dass die Vorrichtung ein Gegenlager, eine konische Hülse und einen Druckstempel aufweist, wobei alle auf einer gemeinsamen Längsachse angeordnet sind und die Hülse und der Druckstempel auf der Längsachse verschiebbar sind, wobei die Hülse zwischen dem Druckstempel und dem Gegenlager angeordnet ist und der Konuswinkel α größer ist als der Klemmkonuswinkel γ eines zu prüfenden Kugelkopfes.

Bevorzugt hat die Hülse einen Konuswinkel α, der mit dem Konuswinkel des Druckstempels identisch ist.

Nachfolgend wird die Erfindung anhand von Figuren weiter erläutert.

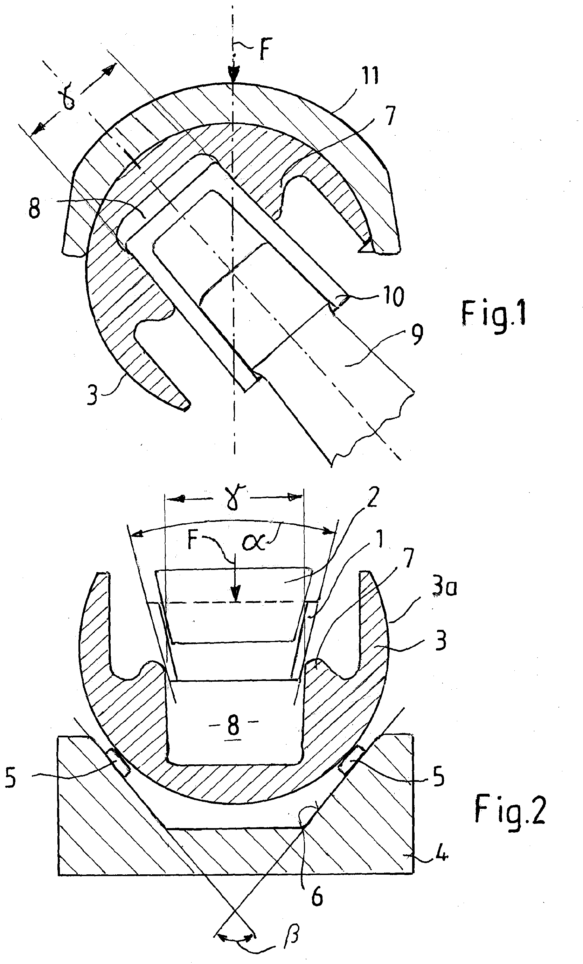

Die Erfindung beschreibt einen Prooftest für den Bereich des Konuseingangs 7 (siehe Figur 1), bei dem durch radial wirkende Kräfte im Bereich des Konuseingangs 7 hohe Spannungen erzeugt werden. Dies kann hydraulisch oder mechanisch erfolgen. Figur 1 zeigt einen Schaft 9 einer Hüftgelenkprothese, der in den Oberschenkelknochen implantiert wird. Auf dem Schaftkopf des Schaftes 9 ist eine Abstandshülse 10 aufgesetzt, die mit ihrer Außenfläche in den Aufnahmeraum 8 des Kugelkopfes 3 eingreift und über eine konische Klemmung mit dem Klemmkonuswinkel γ verankert ist. Der Kugelkopf 3 ist drehbar in eine keramische Gleitschale 11 eingesetzt.

Die mechanische Belastungseinheit des Prooftests besteht aus mindestens zwei Komponenten 1, 2 (siehe Figur 2), die eine Relativverschiebung zueinander ermöglichen. Komponente 1 ist eine geschlossene konische Hülse und Komponente 2 ein konischer Druckstempel, der in die Komponente 2 eingesetzt ist und beim Prüfen axial mit einer Kraft F (siehe Figur 2) in die Komponente 1 gedrückt wird, wodurch der Konuseingang 7 radial mit einer Kraft beaufschlagt wird.

Die Umlenkung der axialen Kraft F in die radiale Richtung wird durch die Relativverschiebung der beiden Komponenten 1 und 2 verstärkt. Die beiden Komponenten 1, 2 sind idealerweise an ihrer Außenfläche konisch gestaltet. Der Konuswinkel α sollte größer als der Klemmkonuswinkel γ des Kugelkopfes sein. Für einen Klemmkonuswinkel γ von 5°42'30" sollte der Konus des Druckstempels im Bereich von 7° bis 30° gewählt werden, idealerweise wird ein Winkel von 18° verwendet. Generell empfiehlt sich für alle Winkelvarianten ein Unterschied zwischen Klemmkonuswinkel γ und Winkel α des Druckstempels von minimal 2° bis maximal 25°.

Das Material der konischen Hülse (Komponente 1) sollte ausreichend duktil sein, um eine Anpassung der Hülse an den Kugelkopf zu gewährleisten. Zusätzlich muss das Material der Hülse ausreichende Festigkeit aufweisen, um der Scherbelastung stand zu halten. Als mögliche Materialien kommen Kupferlegierungen in Frage. Idealerweise besteht die Hülse (Komponente 1) aus Messing.

Der konische Druckstempel (Komponente 2) muss deutlich härter sein als die Hülse. Somit empfiehlt es sich einen Stahl zu verwenden, der gehärtet werden kann.

Idealerweise wird der Druckstempel oberflächengehärtet. Um eine Relativverschiebung zwischen Druckstempel und Hülse sicherzustellen, muss die Oberfläche des konischen Druckstempels glatt sein. Idealerweise wird die Oberfläche des konischen Druckstempels poliert (Ra<0,2). Ausschlaggebend dabei ist, dass die Reibung zwischen Komponente 1 und Komponente 2 deutlich niedriger ist, als zwischen Komponente 1 und dem Kugelkopf. Als Komponente 1 wird immer die Hülse und umgekehrt bezeichnet. Als Komponente 2 wird immer der Druckstempel und umgekehrt bezeichnet.

Für die Lasteinleitung über ein hydraulisches System wird anstelle des konischen Druckstempels die Innenoberfläche der Hülse mit hydraulischem Druck beaufschlagt. Hierfür könnte die Abdichtung über O-Ringe erfolgen. Die Hülse 1 kann sowohl innen als auch außen konisch oder zylindrisch sein.

Zur Durchführung des Prooftests wird der Kugelkopf 3 mit seiner Außenfläche 3a auf ein Gegenlager 4 mit einer konischen Ausnehmung 6 gelegt. Der Winkel der konischen Ausnehmung 6 ist mit β gekennzeichnet. Der Kugelkopf 3 liegt dabei vorteilhaft über einen Ring 5 auf der konischen Ausnehmung 6 des Gegenlagers 4 auf.

Für den Prooftest wird die Komponente 1, d.h. die Hülse, mit eingelegter Komponente 2, d.h. der Druckstempel, auf den Konuseingang 7 des Kugelkopfes 3 gelegt. Der äußere Konuswinkel α der Komponente 1 ist größer als der Klemmkonuswinkel γ des Aufnahmeraums 8 im Kugelkopf 3, so dass die Komponente 1 eine Kraft nur auf den Konuseingang 7 ausübt.

Zum Prüfen wird die Komponente 2 axial mit einer Kraft F in Richtung zum Kugelkopf gedrückt. Da die Komponente 1 nur am Konuseingang 7 des Kugelkopfes 3 aufliegt, wird die Auswirkung einer Krafteinwirkung nur auf diesen Bereich untersucht.

Durch die Relativbewegung der Komponente 2 zur Komponente 1 wird die Kraft auf den Konuseingang 7 verstärkt.

Die Komponente 1, d.h. die Hülse ist bevorzugt auf dem gesamten Umfang geschlossen ausgebildet und ist zum Beispiel nicht geschlitzt. Es hat sich nämlich überraschenderweise herausgestellt, dass es bei geschlitzten Hülsen auch schon bei einer geringeren Krafteinleitung zum Brechen des Kugelkopfes kommt als bei ungeschlitzten Hülsen.

## Patentansprüche

1. Verfahren zum Prüfen von keramischen Kugelköpfen (3) für Hüftgelenksprothesen, die über einen Aufnahmeraum (8) mit einer konischen Seitenfläche mit einem Klemmkonuswinkel γ und einem Konuseingang (7) verfügen und zur Prüfung Bereiche des Aufnahmeraums (8) mit einem Druck beaufschlagt werden, **dadurch gekennzeichnet, dass** ausschließlich der Bereich des Konuseingangs (7) mit einer zur Längsachse des Kugelkopfes (3) senkrecht verlaufenden radialen Kraft beauftragt wird.

2. Verfahren nach Anspruch 1, **dadurch gekennzeichnet, dass** zum Prüfen eine konische Hülse (1) mit einem Konuswinkel α in den Aufnahmeraum (8) gedrückt wird, wobei der Konuswinkel α größer als der Klemmkonuswinkel γ ist.

3. Verfahren nach Anspruch 2, **dadurch gekennzeichnet, dass** der Konuswinkel α im Bereich von 7° bis 30°, bevorzugt 18° gewählt wird und der Unterschied zwischen Klemmkonuswinkel γ und Konuswinkel α zwischen minimal 2° und maximal 25° zu wählen ist.

4. Verfahren nach Anspruch 2 oder 3, **dadurch gekennzeichnet, dass** die Hülse (1) auf ihrer Umfangsfläche geschlossen ist und bevorzugt aus einer Kupferlegierung, bevorzugt Messing besteht.

5. Verfahren nach einem der Ansprüche 2 bis 4, **dadurch gekennzeichnet, dass** ein konischer Druckstempel (2) mit einem mit der Hülse (1) gemeinsamen Konuswinkel α axial mit einer Kraft F in die Hülse (1) gedrückt wird.

6. Verfahren nach Anspruch 5, **dadurch gekennzeichnet, dass** die Reibung zwischen Hülse (1) und Druckstempel (2) niedriger ist als die Reibung zwischen Hülse (1) und Kugelkopf (3).

7. Verfahren nach Anspruch 5 oder 6, **dadurch gekennzeichnet, dass** der Druckstempel (2) aus Stahl besteht, der bevorzugt oberflächengehärtet ist.

8. Verfahren nach einem der Ansprüche 2 bis 4, **dadurch gekennzeichnet, dass** zum Prüfen die Innenoberfläche der Hülse (1) mit einem hydraulischen Druck beaufschlagt wird.

9. Vorrichtung zur Durchführung des Verfahrens nach einem der Ansprüche 1 bis 8, **dadurch gekennzeichnet, dass** die Vorrichtung ein Gegenlager (4), eine konische Hülse (1) und einen Druckstempel (2) aufweist, wobei alle auf einer gemeinsamen Längsachse (12) angeordnet sind und die Hülse (1) und der Druckstempel (2) auf der Längsachse (12) verschiebbar sind, wobei die Hülse (1) zwischen dem Druckstempel (2) und dem Gegenlager (4) angeordnet ist und der Konuswinkel α größer ist als der Klemmkonuswinkel γ eines zu prüfenden Kugelkopfes (3).

10. Vorrichtung nach Anspruch 9, **dadurch gekennzeichnet, dass** die Hülse (1) einen Konuswinkel α hat, der mit dem Konuswinkel des Druckstempels (2) identisch ist.

## Claims

1. A method for checking ceramic ball heads (3) for hip-joint prostheses that have a receiving space (8) with a conical side face with a clamping cone angle γ and a cone inlet (7) and for checking areas of the receiving space (8) are subjected to a pressure, **characterised in that** a radial force that runs perpendicularly to the longitudinal axis of the ball head (3) is applied exclusively to the area of the cone inlet (7).

2. A method according to claim 1, **characterised in that** for checking purposes a conical sleeve (1) with a cone angle α is pressed into the receiving space (8), wherein the cone angle α is greater than the clamping cone angle γ.

3. A method according to claim 2, **characterised in that** the cone angle α is selected in the range of from 7° to 30°, preferably 18°, and the difference between the clamping cone angle γ and the cone angle α is to be selected to be between a minimum of 2° and a maximum of 25°.

4. A method according to claim 2 or 3, **characterised in that** the sleeve (1) is closed on its circumferential surface and preferably consists of a copper alloy, preferably brass.

5. A method according to one of claims 2 to 4, **characterised in that** a conical pressure stamp (2) with a cone angle α which is common to that of the sleeve (1) is pressed axially with a force F into the sleeve (1).

6. A method according to claim 5, **characterised in that** the friction between the sleeve (1) and the pressure stamp (2) is lower than the friction between the sleeve (1) and the ball head (3).

7. A method according to claim 5 or 6, **characterised in that** the pressure stamp (2) consists of steel which is preferably surface-hardened.

8. A method according to one of claims 2 to 4, **characterised in that** for checking purposes the inner surface of the sleeve (1) is subjected to a hydraulic pressure.

9. A device for carrying out the method according to one of claims 1 to 8, **characterised in that** the device has a counter-bearing (4), a conical sleeve (1) and a pressure stamp (2), wherein all are arranged on a common longitudinal axis (12), and the sleeve (1) and the pressure stamp (2) are displaceable on the longitudinal axis (12), wherein the sleeve (1) is arranged between the pressure stamp (2) and the counter-bearing (4), and the cone angle α is greater than the clamping cone angle γ of a ball head (3) that is to be checked.

10. A device according to claim 9, **characterised in that** the sleeve (1) has a cone angle α that is identical with the cone angle of the pressure stamp (2).

## Revendications

1. Procédé de contrôle de têtes sphériques (3) en céramique destinées à des prothèses de hanche, qui disposent d'une cavité de réception (8) dotée d'une surface latérale conique avec un angle de cône de serrage γ et une entrée de cône (7), des parties de la cavité de réception (8) étant soumises à une pression en vue du contrôle, **caractérisé en ce que** seule la partie de l'entrée de cône (7) est soumise à une force radiale s'étendant perpendiculairement à l'axe longitudinal de la tête sphérique (3).

2. Procédé selon la revendication 1, **caractérisé en ce que**, en vue du contrôle, une douille (1) conique avec un angle de cône α est pressée dans la cavité de réception (8), l'angle de cône α étant supérieur à l'angle de cône de serrage γ.

3. Procédé selon la revendication 2, **caractérisé en ce que** l'angle de cône α est choisi dans la plage allant de 7° à 30°, de préférence 18°, et la différence entre l'angle de cône de serrage γ et l'angle de cône α sera choisie entre au minimum 2° et au maximum 25°.

4. Procédé selon la revendication 2 ou 3, **caractérisé en ce que** la douille (1) est fermée sur sa surface périphérique et se compose de préférence d'un alliage de cuivre, de préférence de laiton.

5. Procédé selon l'une des revendications 2 à 4, **caractérisé en ce que** l'on enfonce un poinçon de pression (2) conique, ayant un angle de cône α commun avec la douille (1), axialement avec une force F dans la douille (1).

6. Procédé selon la revendication 5, **caractérisé en ce que** le frottement entre la douille (1) et le poinçon de pression (2) est inférieur au frottement entre la douille (1) et la tête sphérique (3).

7. Procédé selon la revendication 5 ou 6, **caractérisé en ce que** le poinçon de pression (2) est réalisé en acier qui a de préférence subi une trempe superficielle.

8. Procédé selon l'une des revendications 2 à 4, **caractérisé en ce que**, en vue du contrôle, la surface intérieure de la douille (1) est soumise à une pression hydraulique.

9. Dispositif de mise en oeuvre du procédé selon l'une des revendications 1 à 8, **caractérisé en ce que** le dispositif présente un contre-appui (4), une douille (1) conique et un poinçon de pression (2), qui sont tous disposés sur un axe longitudinal (12) commun, la douille (1) et le poinçon de pression (2) pouvant être déplacés sur l'axe longitudinal (12), la douille (1) étant disposée entre le poinçon de pression (2) et le contre-appui (4), et l'angle de cône α étant supérieur à l'angle de cône de serrage γ d'une tête sphérique (3) à contrôler.

10. Dispositif selon la revendication 9, **caractérisé en ce que** la douille (1) présente un angle de cône α qui est identique à l'angle de cône du poinçon de pression (2).
